# EUROPEAN PATENT SPECIFICATION

(11) **EP 4 529 827 B1**
(45) Date of publication and mention of the grant of the patent: **20.05.2026**
(21) Application number: 24202602.9
(22) Date of filing: 25.09.2024
(51) Int. Cl.: A61B 3/028, A61B 3/10

(54) **OPHTHALMOLOGIC APPARATUS**
OPHTHALMOLOGISCHES GERÄT
APPAREIL OPHTALMOLOGIQUE

(30) Priority: 28.09.2023 JP 2023168739
(43) Date of publication of application: 02.04.2025
(73) Proprietor: TOPCON CORPORATION, Tokyo 174-8580 (JP)
(72) Inventor: TATARA, Yoko, Tokyo, 174-8580 (JP); YUKIMORI, Takafumi, Tokyo, 174-8580 (JP); SAIKA, Makoto, Tokyo, 174-8580 (JP); NORO, Ryoka, Tokyo, 174-8580 (JP); SAKAIHARA, Manabu, Tokyo, 174-8580 (JP)
(74) Representative: Louis Pöhlau Lohrentz

(56) References cited:
- ES-A1- 2 631 478
- JP-A- 2007 101 915
- JP-A- 2020 069 201
- US-A1- 2023 414 100

## Description

### FIELD OF THE INVENTION

The present invention relates to an ophthalmologic apparatus.

### BACKGROUND

Techniques for measuring ocular information while the left and right subject eyes binocularly view the fixation targets have been disclosed in some documents such as JP2020-069201A1 and JP2022-038942A1. These conventional ophthalmologic apparatuses measure the ocular information of the subject eyes while the subject eyes view the common target.

### SUMMARY

During the binocular viewing, the subject eyes adjust their focus based on the distance (presentation distance) to the fixation targets and also experience convergence-induced accommodation (referred to as convergence accommodation) to achieve the fusion of the fixation target in the corresponding direction. The convergence accommodation is based on the assumption of binocular viewing, and thus, the adjustment based on the distance occurs simultaneously. Accordingly, the conventional ophthalmologic apparatuses have difficulty in properly measuring the convergence accommodation.

The present invention has been made by considering the above problem. An object of the present invention aims to provide an ophthalmologic apparatus that can properly measure the convergence accommodation. The invention is defined in independent claim 1.

In one embodiment, an ophthalmologic apparatus includes a visual-target presenting portion that is configured to present visual targets to left and right subject eyes, respectively; and an objective measurement optical system that is configured to objectively measure eye characteristics of the left and right subject eyes, respectively. The visual-target presenting portion is configured to present the visual targets while changing a convergence position, where two lines of sight from positions of the left and right subject eyes intersect, with pinhole plates respectively disposed at positions conjugate with pupils of the left and right subject eyes. The objective measurement optical system is configured to obtain the objective refractive values of the left and right subject eyes, respectively, under a condition where the pinhole plates are respectively disposed by the visual-target presenting portion and the visual targets presented at the convergence position are binocularly fused.

Accordingly, such ophthalmologic apparatus can properly measure the convergence accommodation.

### BRIEF DESCRIPTION OF DRAWINGS

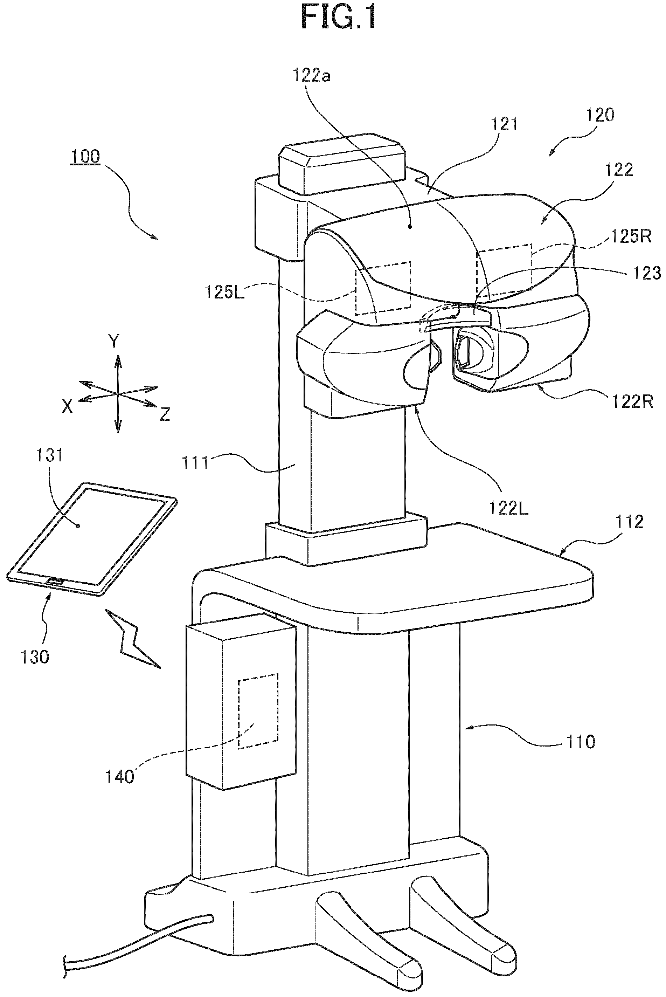

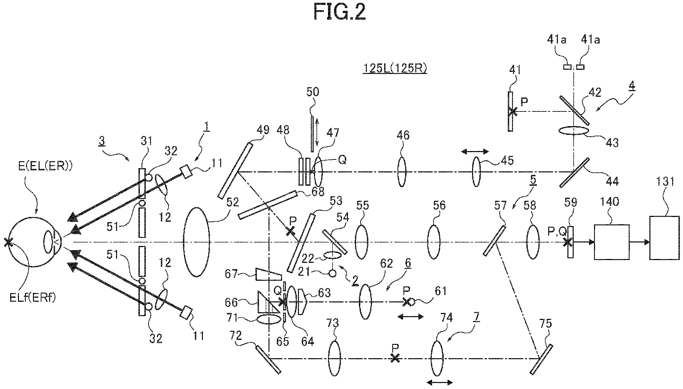

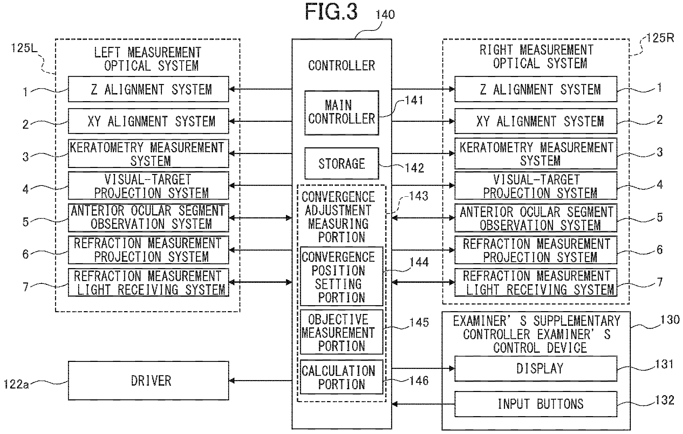

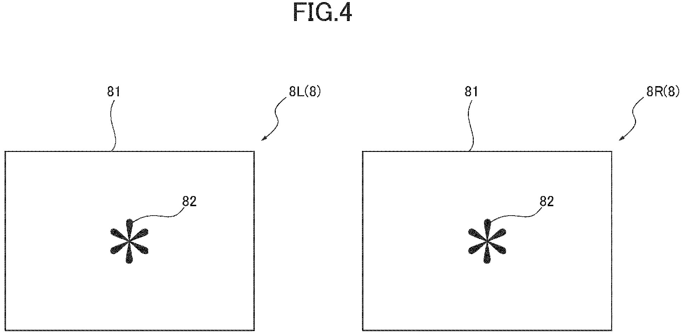

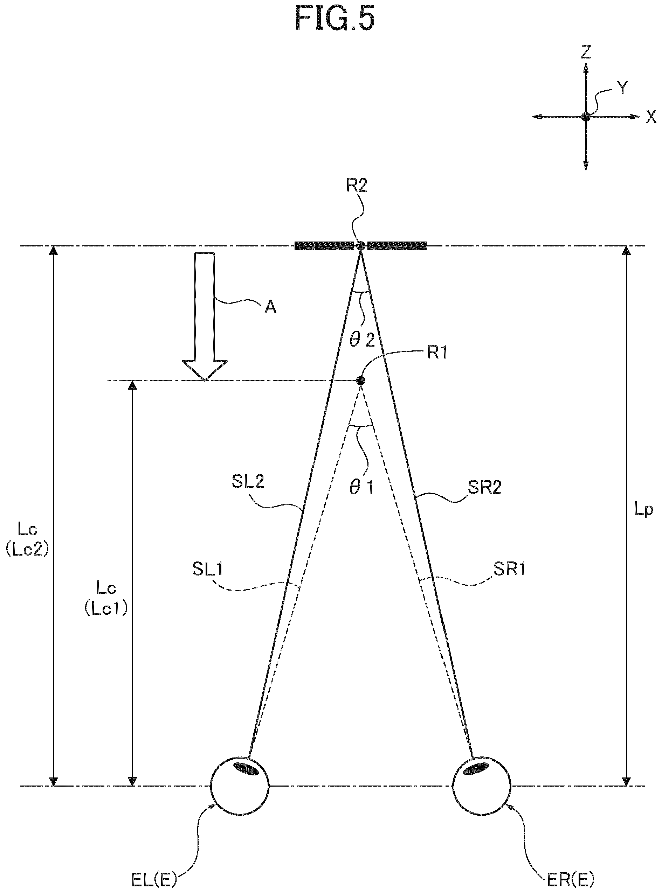

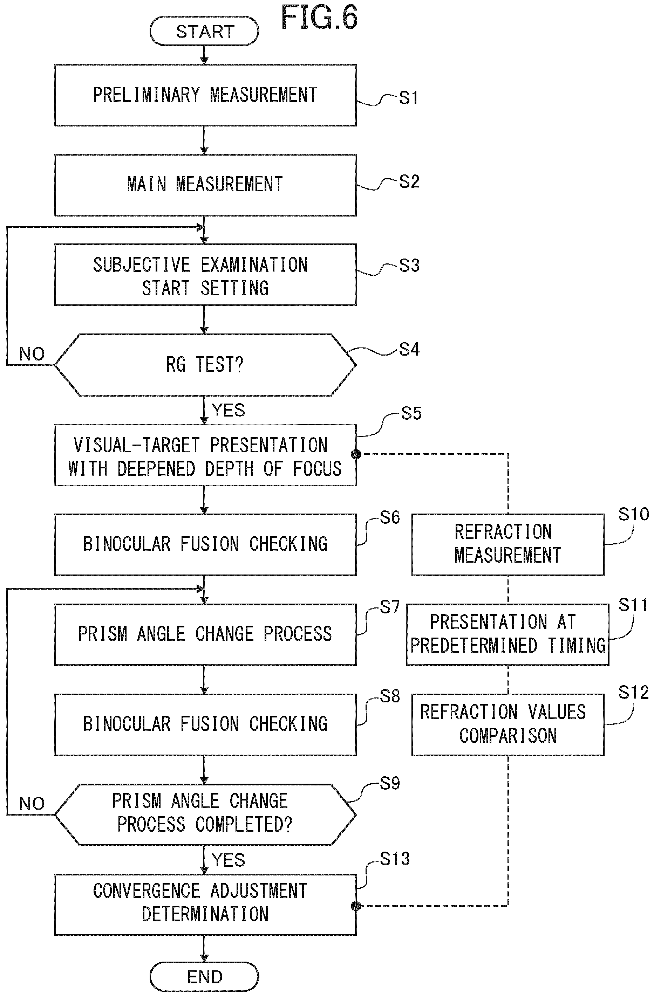

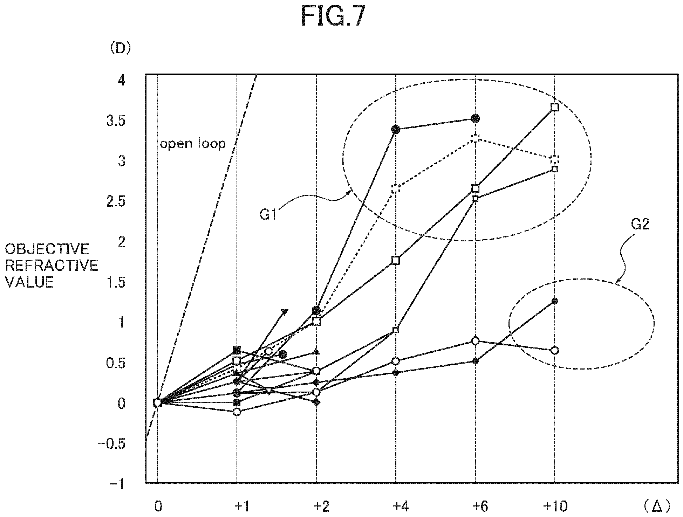

FIG. 1 is an external perspective view illustrating an ophthalmologic apparatus according to a first embodiment. FIG. 2 is a schematic view illustrating an exemplary configuration of a left measurement optical system in the ophthalmologic apparatus according to the first embodiment. FIG. 3 is a block diagram illustrating a control configuration in the ophthalmologic apparatus according to the first embodiment. FIG. 4 is an explanatory view showing left-eye and right-eye visual targets as one example. FIG. 5 is an explanatory view illustrating a presentation distance to the visual targets and a convergence distance to a convergence position where two lines of sight intersect. FIG. 6 is a flowchart illustrating a flow of a process for measuring the convergence accommodation according to the first embodiment. FIG. 7 is a graph showing the relationships between prism angles and objective refractive values in a plurality of examinees, where the average objective refractive value is obtained at each stage of the convergence distance.

### DETAILED DESCRIPTION

For the use of plural and/or singular terms herein, those having skill in the art can translate from the plural to the singular and/or from the singular to the plural as is appropriate to the context and/or application. The various singular/plural permutations may be expressly set forth herein for the sake of clarity.

An embodiment of an ophthalmologic apparatus according to the present invention will be described with reference to FIGS. 1 to 7.

### EMBODIMENT

An example of an embodiment of an ophthalmologic apparatus and a method for examining subject eyes will be described based on a first embodiment illustrated in the drawings.

As shown in FIG. 1, an ophthalmologic apparatus 100 is a both-eye open type apparatus capable of simultaneously measuring the eye characteristics of both eyes at the same time with an examinee opening both eyes. The ophthalmologic apparatus 100 may also individually measure the eye characteristics of each eye at a time by shielding one of the eyes or turning off a fixed target for one of the eyes. The ophthalmologic apparatus 100 is an objective measurement apparatus with subjective measurement functions and the objective measurement apparatus includes a visual target presenting function, a phoropter function, and an automatic refraction and keratometry measurement function. The ophthalmologic apparatus 100 allows the examiner to objectively and subjectively measure the eye characteristics of the subject eyes with objective and subjective examinations.

The objective examinations executed by the ophthalmologic apparatus 100 include the measurement for obtaining the eye characteristics and the photographing for obtaining the image of the subject eye E. The objective examinations include the refractive power measurement (refraction measurement), the corneal shape measurement (keratometry measurement), the intraocular pressure measurement, the ocular fundus photographing, the tomogram imaging using optical coherence tomography (OCT) (OCT imaging), and the measurement using OCT. The subjective examination presents a target to an examinee and measures information (eye characteristics) on the subject eye based on the responses from the examinee to the presented target. The subjective examinations include subjective refraction measurements such as the far-point test, the intermediate-point test, the near-point test, the contrast test, and the glare test, and the visual field test.

The ophthalmologic apparatus 100 includes a support base 110, a measurement unit 120, an examiner's control device 130, and a controller 140. This embodiment uses a vertical direction (a Y axis direction), a front and back direction (a Z axis direction i.e., near side and far side directions when FIG. 1 is viewed from the front), and a left and right direction (an X axis direction, i.e., left and right direction when FIG. 1 is viewed from the front) that are orthogonal to the vertical direction and the front and back direction. The upper side of the vertical direction is defined as the positive side of the Y axis direction, the examinee's side in the front and back direction (right near side when FIG. 1 is viewed from the front) is defined as the positive side of the Z axis direction, and the right far side in the left and right directions when FIG. 1 is viewed from the front is defined as the positive side of the X axis direction.

The support base 110 includes a column 111 rising from a floor and an optometric table 112 supported by the column 111. The optometric table 112 is a base for supporting a posture of the examinee. The optometric table 112 is also used to place a device and a tool for use in optometry, such as the examiner's control device 130, thereon. The optometric table 112 may be fixed at a position (height position) in the Y axis direction or may be supported on the column 111 to adjust the position (height position) of the optometric table 112 in the Y axis direction.

The measurement unit 120 includes an arm 121, a measurement head 122, and a forehead rest 123. One end of the arm 121 is supported by an end portion of the column 111, and the other end of the arm 121 extends toward a near side (the examinee's side) from the column 111 in the Z axis direction. The measurement head 122 is attached to an end of the arm 121. The measurement head 122 is thereby hung on the column 111 through the arm 121 above the optometric table 112. The arm 121 is configured to move in the Y axis direction relative to the column 111. The arm 121 may be configured to move in the X axis and Z axis directions relative to the column 111.

The measuring head 122 measures the eye characteristics of subject eyes E (see FIGS. 2 and 4). The measuring head 122 includes a driver 122a, the left and right measuring head portions 122L, 122R, which are provided under the driver 122a, and the forehead rest 123. The left and right measuring head portions 122L, 122R are configured to measure the left and right subject eyes EL, ER of the examinee, respectively. The driver 122a allows each of the left and right measuring head portions 122L, 122R to move horizontally (in the X axis direction) and vertically (in the Y axis direction) and to rotate about the X axis and the Y axis.

The left measuring head portion 122L includes a left measurement optical system 125L. The left measurement optical system 125L is configured to present a visual target 8 (see FIG. 4) to the left subject eye EL of the examinee and to measure the eye characteristics of the left subject eye EL under a condition that is set at any spherical power (corrected power). The right measuring head portion 122R includes a right measurement optical system 125R. The right measurement optical system 125R is configured to present a visual target 8 to the right subject eye ER of the examinee and to measure the eye characteristics of the right subject eye ER under a condition that is set at any spherical power (corrected power). Detailed configurations of the left and right measurement optical systems 125L, 125R are described below.

The forehead rest 123 is provided in the measurement unit 120 and is disposed between the left and right measuring head portions 122L, 122R. The forehead rest 123 supports the face of the examinee when the examinee places the forehead of his or her face in contact with the forehead rest 123 during the measurement of the eye characteristics. In other words, the examinee facing towards the optometric table 112 can press his or her forehead against the forehead rest 123 so that he or she can stabilize the direction and the position of the face. The position of the forehead rest 123 in the height direction can be adjusted by moving the arm 121 in the Y axis direction relative to the column 111.

The examiner's control device 130 is an information processing device that receives input operations by the examiner and outputs control signals to the controller 140. The examiner's control device 130 can be implemented with a portable device such as a tablet terminal or a smartphone, which can be separated from the measurement unit 120 so that the examiner can carry the examiner's control device 130. The examiner's control device 130 may be implemented with a personal computer such as a laptop personal computer or a desktop personal computer. The examiner's control device 130 may be implemented with a dedicated controller provided in the ophthalmologic apparatus 100. The examiner's control device 130 can exchange information with the controller 140 via wireless communication and/or network communication.

The examiner's control device 130 includes a display 131, input buttons 132, and an operation controller (not shown). The display 131 is a touch panel display provided on the examiner's control device 130. The display 131 may be configured to display buttons and/or icons such as the input buttons 132 (see FIG. 3). The operation controller is a microcomputer built in the examiner's control device 130. The operation controller is configured to control images displayed on the display 131 based on the measurement results and/or the detection results sent from the controller 140. The operation controller outputs the control signals to the controller 140 according to the operation to the input buttons 132 and other buttons or icons.

The detailed configurations of the left and right measurement optical systems 125L, 125R will be described with reference to FIG. 2. The left and right measurement optical systems 125L, 125R have the same configuration. Accordingly, only the left measurement optical system 125L is described and the description of the right measurement optical system 125R is omitted in this specification.

The left measurement optical system 125L is an optical system that presents the visual target 8 (see FIG. 4) to the left subject eye EL and examines it. The left measurement optical system 125L includes a Z alignment system 1, an XY alignment system 2, a keratometry measurement system 3, a visual-target projection system 4, an anterior ocular segment observation system 5, a refraction measurement projection system 6, and a refraction measurement light receiving system 7. In the following description, an ocular fundus conjugate position P and a pupil conjugate position Q are used. The ocular fundus conjugate position P means position optically and substantially conjugate with an ocular fundus ELf of the left subject eye EL, which can include an exact position optically conjugate with the ocular fundus ELf and any proximal positions near the exact position, in an aligned state. The pupil conjugate position Q means position optically and substantially conjugate with a pupil of the left subject eye EL, which can include an exact position optically conjugate therewith and any proximal positions near the exact position, in an aligned state.

The Z alignment system 1 is configured to project light (infrared light in the first embodiment) onto the left subject eye EL for the alignment purpose in the direction of the optical path (front or back direction) of the anterior ocular segment observation system 5. In the Z alignment system 1 includes a Z alignment light source 11 which emits light. The light from the Z alignment light source 11 is turned into a parallel light flux by using the projecting lens 12 and then is projected onto the cornea of the left subject eye EL through an alignment hole formed through a keratometric plate 31. Based on a bright spot projected onto the cornea, the controller 140 or the examiner moves the left measurement optical system 125L in the Z axis direction by controlling the driver 122a such that the ratio of the distance between two spot images by the Z alignment light source 11 on the imaging element 59 of the anterior ocular segment observation system 5 and the diameter of the keratometric ring image should fall in a predetermined range. This places the left measurement optical system 125L at a proper position in the optical axis direction relative to the left subject eye EL.

The XY alignment system 2 is configured to illuminate light (infrared light in the first embodiment) onto the left subject eye EL for the alignment purpose in the X axis and Y axis directions orthogonal to the optical axis (Z axis) of the anterior ocular segment observation system 5. The XY alignment system 2 includes an XY alignment light source 21 and a projecting lens 22 in an optical path branched from the anterior ocular segment observation system 5 by using the half mirror 54. The XY alignment system 2 proceeds the light emitted from the XY alignment light source 21 to the anterior ocular segment observation system 5 through the projecting lens 22. Then, the XY alignment system 2 reflects the light with the half mirror 54 and then projects the reflected light onto the left subject eye EL through the anterior ocular segment observation system 5. The reflected light from the cornea of the left subject eye EL is guided to the imaging element 59 through the anterior ocular segment observation system 5. Thereby, the left measurement optical system 125L is placed at a proper position in the X axis and Y axis directions relative to the left subject eye EL. The alignment method in each of XYZ directions is not limited to the method using the Z alignment system 1 and the XY alignment system 2. The alignment method may be ones capable of measuring the position of the subject eye E, for example, by using a stereo camera in the ophthalmologic apparatus 100.

The image (bright spot image) based on the reflected light from the cornea is formed in a manner to overlap the anterior ocular segment image. The controller 140 is configured to control the display of the anterior ocular segment image including the bright spot image and the alignment mark on the display 131. With the manual XY alignment, the examiner operates the examiner's control device 130 to control the driver 122a to move the left measurement optical system 125L in the X axis and Y axis directions and to guide the bright spot image within the alignment mark. With the automatic XY alignment, the controller 140 controls the driver 122a to cancel the displacement of the bright point image relative to the alignment mark and to move the left measurement optical system 125L in the X axis and Y axis directions.

The keratometry measurement system 3 is configured to project a ring-shaped light flux (infrared light) onto the cornea for measuring the shape of the cornea of the left subject eye EL. The keratometry measurement system 3 includes the keratometric plate 31 disposed between the objective lens 52 and the left subject eye EL, and a keratometric ring light source 32 provided on the back side (side of the objective lens 52) of the keratometric plate 31. The keratometry measurement system 3 projects the ring-shaped light flux on the cornea of the left subject eye EL by illuminating the keratometric plate 31 with the light from the keratometric ring light source 32. The imaging element 59 detects the reflected light (keratometric ring image) from the cornea of the left subject eye EL together with the anterior ocular segment image. The controller 140 calculates the cornea shape parameter representing the shape of the cornea with a known calculation based on the keratometric ring image.

The visual-target projection system 4 presents various visual targets, such as fixation targets and visual targets for the subjective examination, to the left subject eye EL. The visual-target projection system 4 includes a display 41, a half mirror 42, a relay lens 43, a reflective mirror 44, a focusing lens 45, a relay lens 46, a field lens 47, a variable cross-cylinder lens (VCC) 48, a reflective mirror 49, and a pinhole plate 50. The visual-target projection system 4 shares a dichroic mirror 68 with the refraction measurement projection system 6. In addition, the visual-target projection system 4 shares a dichroic mirror 53 and the objective lens 52 with the anterior ocular segment observation system 5. The visual-target projection system 4 further includes at least two glare light sources 41a, 41a, which illuminate the left subject eye EL with glare light, at a position around the optical axis and on an optical path different from the optical path to elements such as the display 41 which displays the visual target 8.

The display 41 functions as a visual-target presenting portion that presents the visual target 8. The display 41 is configured to display the fixation target or the point-like visual target as the visual target for fixing a line of sight during the objective examination or fogging to the left subject eye EL. The display 41 is also configured to display the subjective examination visual target for subjectively examining eye characteristics (e.g., visual acuity value, far-point power, and near-point power) of the left subject eye EL. The display 41 may be implemented with a liquid crystal display (LCD) is used for the display 41. The display 41 may be implemented with another display such as an organic electroluminescence (EL) display. Then, the visual target 8 to be projected on the subject eye E can be arbitrarily created by using a still image or a moving image, which can be used as a chart icon that can be selected on a chart page. The display 41 is configured to display the created visual target 8 of the still or moving image selected from the chart page. The display 41 is provided at the fundus conjugate position P on the optical path of the visual target projection system 4.

The visual-target projection system 4 reflects the light from the display 41 with the half mirror 42, transmits the light through the relay lens 43, to be reflected by the reflective mirror 44, and then to be transmitted through the focusing lens 45. Subsequently, the visual-target projection system 4 transmits the light through the relay lens 46, aligns its travel direction with the field lens 47, transmits the light through the VCC 48, reflects the light with the reflective mirror 49, transmits the light through the dichroic mirror 68, and then reflects the light with the dichroic mirror 53. Next, the visual-target projection system 4 irradiates the light reflected by the dichroic mirror 53 onto the ocular fundus Elf through the objective lens 52.

The controller 140 controls a drive motor (not shown) to drive the focusing lens 45 to move forward or backward in the optical axis direction. The controller 140 moves the focusing lens 45 toward the left subject eye EL to displace the spherical power of the left subject eye EL to the negative diopter side (-D side). Also, the controller 140 moves the focusing lens 45 in a direction away from the left subject eye EL to displace the spherical power of the left subject eye EL to the positive diopter side (+D side). Furthermore, the controller 140 controls the forward or backward movement of the focusing lens 45 to change the presentation distance Lp from the left subject eye EL to the visual-target presentation position. The focusing lens 45 is controlled to be moved together with a refraction measurement light source 61 of the refraction measurement projection system 6 and a focusing lens 74 of the refraction measurement light receiving system 7.

For the subjective examination, the controller 140 controls the focusing lens 45 to move in the optical axis direction based on the result of the objective examination, thereby controlling the presentation distance Lp and the spherical power of the left subject eye EL. The controller 140 controls the display 41 to display a predetermined visual target 8, which has been selected by the examiner, etc., to present the predetermined visual target 8 to the examinee at a predetermined presentation distance Lp relative to the left subject eye EL adjusted to having a predetermined spherical power. When the examinee responds to the subjective examination about the visual target 8, the controller 140 receives the input of the response. For example, in the visual acuity measurement, the controller 140 selects the next visual target 8 based on the examinee's response to the visual target such as the Landolt ring, displays the selected next visual target 8 to the examinee, and determines the visual acuity value by repeating this process. Accordingly, the visual-target projection system 4 functions as a subjective examination system.

The visual-target projection system 4 includes the pinhole plate 50 at the pupil conjugate position Q. **In** the first embodiment, the pinhole plate 50 is provided between the field lens 47 and the VCC 48. This pinhole plate 50 is formed by making a through hole in a plate member. The controller 140 controls the pinhole plate 50 to be inserted into and removed from the optical path of the visual-target projection system 4. When the pinhole plate 50 is inserted into the optical path, the through hole is positioned on the optical axis. Inserting the pinhole plate 50 into the optical path during the subjective examination allows for a pinhole test that determines if the left subject eye EL can be corrected with glasses. Furthermore, the pinhole plate 50 is also inserted into the optical path to measure the convergence accommodation, which is described later. The pinhole plate 50 is not limited to the configuration in the first embodiment. The pinhole plate 50 may be provided at a position that is substantially conjugate with the pupil of the left subject eye EL in the optical path.

The anterior ocular segment observation system 5 is configured to observe the anterior ocular segment of the left subject eye EL and capture images thereof. The anterior ocular segment observation system 5 includes an anterior ocular segment illumination light source 51 that is configured to illuminate an illumination light (infrared light in the first embodiment) onto the anterior ocular segment of the left subject eye EL. The anterior ocular segment observation system 5 allows the light reflected from the anterior ocular segment of the left subject eye EL to pass through the objective lens 52, to be transmitted through the dichroic mirror 53 and the half mirror 54, to pass through the relay lens 55 and the relay lens 56, and to be transmitted through the dichroic mirror 57. The anterior ocular segment observation system 5 allows the light to form an image on the imaging surface of the imaging element 59 by using the imaging lens 58. The imaging surface of the imaging element 59 is at the pupil conjugate position Q. The imaging element 59 captures images to output a signal at a predetermined rate and also outputs the image signal to the controller 140. The controller 140 controls the display 131 of the examiner's control device 130 to display the anterior ocular segment image (moving image), which is based on the image signal output from the imaging element 59, on the display 131.

The refraction measurement projection system 6 and the refraction measurement light receiving system 7 constitute an objective measurement optical system for use in the objective refraction measurement that objectively measures the refraction values as the eye characteristics of the left subject eye EL. Hereinafter, the refractometry projection system 6 and the refraction measurement light receiving system 7 are referred to as objective measurement optical systems 6, 7. The refraction measurement projection system 6 projects a ring-shaped light flux (infrared light) for objective measurement from the refraction measurement light source 61 onto the ocular fundus Ef. The refraction measurement light receiving system 7 receives the return light from the left subject eye EL of the ring-shaped light flux.

**In** the first embodiment, the refraction measurement light source 61 is implemented with a super luminescent diode (SLD) light source, which is a high-intensity light source with an emission diameter less than a predetermined size. The refraction measurement light source 61 is configured to move in the optical axis direction in conjunction with the focusing lens 45 and the focusing lens 74 and is disposed at the ocular fundus conjugate position P. A ring diaphragm 65 is a transmissive portion formed in a ring-shaped and is disposed at the pupil conjugate position Q. The focusing lens 74 is configured to move in the optical axis direction in conjunction with the refraction measurement light source 61 and the focusing lens 45. The focusing lens 74 may be a known variable focusing lens, which can change its focal position under the control of the controller 140. The optical system of the refraction measurement light receiving system 7 disposes the imaging surface of the imaging element 59 at the ocular fundus conjugate position P.

The refraction measurement projection system 6 allows the light emitted from the refraction measurement light source 61 to pass through a relay lens 62 and to enter onto a cone surface of a conical prism 63. The refraction measurement projection system 6 allows the light entered onto the cone surface to be deflected, to be emitted from the bottom surface of the conical prism 63, and to pass through a field lens 64 and a ring diaphragm 65 (its transmissive portion). The refraction measurement projection system 6 allows the light (ring-shaped light flux) to be reflected off a reflection surface of a holed prism 66, to pass through a rotary prism 67, and to be reflected off a dichroic mirror 68. The refraction measurement projection system 6 allows the reflected light to be reflected off the dichroic mirror 53, to pass through the objective lens 52, and to be projected onto the left subject eye EL.

It is preferable to dispose the conical prism 63 at a position as close as possible to the pupil conjugate position Q. The conical prism 63 may include a ring diaphragm 65 attached to its bottom surface facing the field lens 64. In this case, for example, the bottom surface of the conical prism 63 may be deposited with a light shielding film to form a ring-shaped light transmitting portion. Alternatively, the ring diaphragm 65 may be on the conical surface side of the conical prism 63.

The field lens 64 may include the ring diaphragm 65 affixed to the lens surface on the side of the left subject eye EL, for example. In this case, a light shielding film may be deposited on the lens surface of the field lens 64 so that a ring-shaped transmissive portion is formed. The refraction measurement projection system 6 may have a configuration in which the field lens 64 is omitted. The ring diaphragm 65 may be formed with the light transmitting portion having a shape corresponding to a predetermined measurement pattern, at a position eccentric to (offset from) the optical axis of the refractometry projection system 6. The number of the light transmitting portions may be one or more than two. The rotary prism 67 is used for averaging the light amount distribution of the ring-shaped luminous flux with respect to the disease site and the blood vessel of the fundus ELf and reducing speckle noise caused by the light source.

The refraction measurement light receiving system 7 allows the return light of the ring-shaped light flux projected onto the ocular fundus Elf to pass through the objective lens 52 and to be reflected off the dichroic mirror 53 and the dichroic mirror 68. The refraction measurement light receiving system 7 allows the reflected return light to pass through the rotary prism 67, the hole of the holed prism 66, and the relay lens 71, to be reflected off the reflective mirror 72, and to pass through the relay lens 73 and the focusing lens 74. The refraction measurement light receiving system 7 allows the light, which has passed therethrough, to be reflected off a reflective mirror 75, to pass through the dichroic mirror 57, and to form an image on the imaging surface of the imaging element 59 with the imaging lens 58.

The controller 140 calculates the parameter of the ocular refractive power by using any known calculation, based on the output from the imaging element 59. The parameter of the ocular refractive power includes refractive value (refractive power), spherical power, astigmatism power, and astigmatic axis angle of the left and right subject eyes EL, ER. Based on the control signals from the examiner's control device 130, the controller 140 comprehensively controls the left and right measurement optical systems 125L, 125R (each of which includes the refraction measurement projection system 6, the refraction measurement light receiving system 7, the visual target projection system 4), and the measurement unit 120 (which includes the driver 122a). The controller 140 sends the measurement results of the eye characteristics of the left and right subject eyes EL, ER measured by the measuring head 122 to the examiner's control device 130.

Next, the configuration of the controller 140 is described with reference to FIG. 3. As shown in FIG. 3, the controller 204 includes a main controller 141, a storage 142, and a convergence accommodation measuring portion 143.

The main controller 141 is configured to control the adjustment of light intensity and the on/off switching of the Z alignment light source 11 of the Z alignment system 1, the XY alignment light source 21 of the XY alignment system 2, and the keratometric ring light source 32 of the corneal ring measurement system 3. The main controller 141 controls the on/off switching of the visual target 8 displayed on the display 41 of the visual-target projection system 4 and the selection of the visual target 8. The main controller 141 controls the switching between the insertion of the pinhole plate 50 of the visual-target projection system 4 into the optical path and the retraction of the pinhole plate 50 therefrom. The main controller 141 controls the adjustment of light intensity and the on/off switching of the anterior ocular segment illumination light source 51 of the anterior ocular segment observation system 5. The main controller 141 controls the changing of the exposure time and the detection sensitivity of the imaging element 59 of the anterior ocular segment observation system 5. The main controller 141 controls the adjustment of light intensity and the on/off switching of the refraction measurement light source 61 of the refraction measurement projection system 6. The main controller 141 controls changing the rotation speed and the on/off switching of the rotation movement of the rotary prism 67 of the refraction measurement projection system 6. The main controller 141 controls the position changing of the relay lens 56 of the anterior ocular segment observation system 5 in the optical axis direction. The main controller 141 controls the movement of the focusing lens 45 of the visual-target projection system 4, the refraction measurement light source 61 of the refraction measurement projection system 6, and the focusing lens 74 of the refraction measurement light receiving system 7 in conjunction with each other in the optical axis direction. The main controller 141 controls the processes of writing data to the storage 142 and reading the data from the storage 142.

The storage 142 stores various data. The stored data include information and data such as the measurement information acquired by the keratometry measurement system 3, the measurement information acquired by the objective measurement optical systems 6, 7, the data of the images acquired by the imaging element 59, and the subject eye information. The subject eye information includes information regarding examinees, such as a patient's IDs and names, and information regarding the left and right subject eyes EL, ER such as identification information of left and right eyes. The storage 142 stores the measurement information acquired by the keratometry measurement system 3 when the ophthalmologic apparatus performs the keratometry measurement of the left and right subject eyes EL, ER. The storage 142 stores the measurement information acquired by the objective measurement optical systems 6, 7 when the ophthalmologic apparatus performs the refraction measurement of the left and right subject eyes EL, ER. The storage 142 may be used as a working memory of the calculation process of the corneal shape parameter and the calculation process of the refraction values of the left and right subject eyes EL, ER. The storage 142 stores various programs and data for operating the ophthalmologic apparatus 100.

The convergence accommodation measuring portion 143 is configured to measure the convergence accommodation during the stereoscopic vision of the left and right subject eyes EL, ER (see FIG. 6). The convergence accommodation measuring portion 143 includes a convergence position setting portion 144, an objective measurement portion 145, and a calculation portion 146.

As shown in FIG. 4, the convergence position setting portion 144 is configured to present the left-eye visual target 8L and the right-eye visual target 8R at a visual target position away from the left and right subject eyes EL, ER, respectively, by a certain presentation distance Lp (e.g., infinity) with the left and right eyes EL, ER being in a state of increased depth of focus. As shown in FIG. 1, the ophthalmologic apparatus 100 includes the left measuring head portion 122L including the left measurement optical system 125L corresponding to the left subject eye EL, and the right measuring head portion 122R including the right measurement optical system 125R corresponding to the right subject eye ER. The left-eye and right-eye visual targets 8L, 8R are set on the displays 41 (visual-target presenting portions) of the visual-target projection systems 4 in the left and right measuring head portions 122L, 122R, respectively. The left measurement optical system 125L projects the left-eye visual target 8L onto the left subject eye EL and the right measurement optical system 125R projects the right-eye visual target 8R onto the right subject eye ER. Accordingly, the convergence position setting portion 144 presents the left-eye and right-eye visual targets 8L, 8R, respectively.

An example of the left-eye and right-eye visual targets 8L, 8R is described with reference to FIG. 4. FIG. 4 shows the left-eye visual target 8L on the left side and the right-eye visual target 8R on the right side. As shown on the left side of FIG. 4, the left-eye visual target 8L includes a square-shaped visual target frame 81 such as a liquid crystal frame, and an asterisk mark 82 at its center. As shown on the right side of FIG. 4, the right-eye visual target 8R includes a square-shaped visual target frame 81, such as a liquid crystal frame, and an asterisk mark 82 at its center. Each of the left-eye and right-eye visual targets 8L, 8R include the asterisk mark 82 of the same design, size, and line width at the center of the visual target frame 81. The design, size, and line width of the asterisk mark 82 are determined to provide an appropriate fusion stimulus to the left and right subject eyes EL, ER during the binocular fusion where the images projected onto the retina of the left and right subject eyes EL, ER are integrated into a single image when viewed binocularly.

The asterisk mark 82 may have its size and radial line width set as appropriate. The left-eye and right-eye visual targets 8L, 8R may include other figures instead of the asterisk mark 82.

As a result, the convergence position setting portion 144 enables the left-eye and right-eye visual targets 8L, 8R to be viewed as a single image, allowing for the binocular fusion. The convergence position setting portion 144 adjusts the convergence distance Lc by controlling the prism angles directed from the left and right subject eyes EL, ER toward the left-eye and right-eye visual targets 8L, 8R. The relationship between the presentation distance Lp and the convergence distance Lc is described below with reference to FIG. 5.

The presentation distance Lp refers to the distance along the Z axis direction from the left and right subject eyes EL, ER to the position where the visual targets 8 are presented. In an example shown in FIG. 5, the visual targets 8 are presented at a convergence position R2, which is described later. The presentation distance Lp is set by adjusting the optical distance to the display 41 in the visual-target projection system 4 (mainly the position of the focusing lens 45 in the first embodiment). Furthermore, the presentation distance Lp can also be referred to as the focal adjustment distance when the left and right subject eyes EL, ER are focused on the visual targets 8. In the first embodiment, the presentation distance Lp is defined as the distance to the visual targets 8 from a straight line connecting the left and right subject eyes EL, ER. However, the presentation distance Lp is not limited to that in the first embodiment. The presentation distance Lp may be the distance to the visual target 8 from each of the subject eyes EL, ER.

The convergence distance Lc refers to the distance along the Z axis direction from the position of the left and right subject eyes EL, ER to the convergence position where the two lines of sight intersect. In an example shown in FIG. 5, the convergence distance Lc includes a convergence distance Lc1 and a convergence distance Lc2. The convergence distance Lc1 is the distance along the Z axis direction from the position of the left and right subject eyes EL, ER to a convergence position R1 where two lines of sight SL1, SR1 intersect. The convergence distance Lc2 is the distance along the Z axis direction from the position of the left and right subject eyes EL, ER to a convergence position R2 where two lines of sight SL2, SR2 intersect. In the first embodiment, the convergence distance Lc is defined as the distance from the straight line connecting the left and right subject eyes EL, ER to the convergence position R where the two lines of sight intersect. However, the convergence distance Lc is not limited to that in the first embodiment. The convergence distance Lc may be the distance from each of the subject eyes EL, ER to the convergence position R.

Here, suppose that the convergence distance Lc is changed from the convergence distance Lc2 to the convergence distance Lc1 as shown by the arrow A in FIG. 5. Changing the convergence distance from the convergence distance Lc2 to the convergence distance Lc1 results in a change in the convergence angle from a convergence angle θ2 defined by the intersecting lines of sight SL2, SR2 to a convergence angle θ1 defined by the intersecting lines of sight SL1, SR1. Accordingly, changing the convergence angle from the convergence angle θ2 to the convergence angle θ1 induces an eyeball movement in a direction that causes the left and right subject eyes EL, ER to move towards each other, which provides the convergence stimulus.

The convergence position setting portion 144 presents the left-eye and right-eye visual targets 8L, 8Rb with the pinhole plate 50 inserted into the pupil conjugate position Q in the optical path in the visual-target projection systems 4 of the left and right measuring head portions 122L, 122R. Thus, the depth of focus of the left and right subject eyes EL, ER is increased in the visual-target projection systems 4, thereby the corresponding left-eye and right-eye visual targets 8L, 8R can be clearly seen by the left and right subject eyes EL, ER to a certain extent even without adjustment according to the presentation distance Lp. Accordingly, the adjustment function of the left and right subject eyes EL, ER does not operate due to the change in the presentation distance Lp even when the convergence distance Lc2 is changed to the convergence distance Lc1. Thus, the objective refractive values of the left and right subject eyes EL, ER should not change in principle even during the objective measurement of the left and right subject eyes EL, ER. However, in fact, the adjustment function of the left and right subject eyes EL, ER operates to change the lens curvature when induced by the convergence stimulus, which results in a variation in the objective refractive values of the left and right subject eyes EL, ER during the objective measurement. Therefore, the convergence accommodation measuring portion 143 can place the left and right subject eyes EL, ER into a condition where only the convergence stimulus is provided by fusing the left-eye and right-eye visual targets 8L, 8R with the pinhole plate 50 inserted.

The convergence position setting portion 144 controls the angles of sight from the left and right subject eyes EL, ER toward the left-eye and right-eye visual targets 8L, 8R, thereby changing the convergence distance Lc from the position of the left and right subject eyes EL, ER to the convergence position where the two lines of sight intersect. The convergence position setting portion 144 controls the left and right measuring head portions 122L, 122R to pivot in opposite directions around respective rotation axes extending through the left and right subject eyes EL, ER in the Y axis direction, thereby changing their rotation angles and thus adjusting the angles of sight (i.e., rotation angle control). The convergence position setting portion 144 commands the driver 122a to operate the rotation angle control.

With the rotation angle control in effect, the left and right measuring head portions 122L, 122R adjust the angles of the optical axes of the visual-target projection systems 4, which are directed from the left and right subject eyes EL, ER toward the displays 41 that present the visual targets 8. Thus, the rotation angle control is equivalent to adding a prism lens at any position along the optical axis of the visual-target projection system 4 and controlling the deviation angle using the prism lens. Accordingly, the angle of sight is hereinafter referred to as the "prism angle," which represents the prism diopter of the prism lens. For example, when a prism lens deflects a beam of light that has passed therethrough by 1 cm on a vertical screen positioned 1 m away, the prism diopter is called 1 prism diopter (1 Δ), and the angle of deviation in this case becomes 0.57 degrees. Here, the following description proceeds by defining the prism diopter in a direction that brings the convergence distance Lc closer to the subject eyes, causing the eyes to rotate inward, as positive, and the prism diopter in a direction that moves the convergence distance Lc farther from the subject eyes, causing the eyes to rotate outward, as negative. The convergence position setting portion 144 creates a condition in which the convergence distance Lc changes incrementally from a far position to a near position by controlling the prism angle incrementally, with the left and right subject eyes EL, ER directed toward the left-eye and right-eye visual targets 8L, 8R, respectively.

The objective measurement portion 145 executes the process in parallel with that in the convergence position setting portion 144. The objective measurement portion 145 measures the eye characteristics of the left and right subject eyes EL, ER using the objective measurement optical systems 6, 7 when the convergence distance Lc is changed, thereby obtaining the objective refractive values as the objective measurement information. Here, as mentioned above, the process in the convergence position setting portion 144 sets a condition in which the convergence distance Lc changes incrementally. Therefore, the objective measurement portion 145 may measure the eye characteristics of the left and right subject eyes EL, ER a predetermined number of times at each stage of changing the convergence distance Lc and average the objective refraction values obtained at each stage to obtain an average objective refraction value as the objective measurement information.

The calculation portion 146 determines the convergence accommodation of the left and right subject eyes EL, ER based on the objective refractive values (refraction values) obtained by the objective measurement portion 145 at each convergence distance Lc, that is, for each prism diopter set by the convergence position setting portion 144. Here, the convergence position setting portion 144 creates a condition where the left and right subject eyes EL, ER are exposed only to the convergence accommodation stimulus by presenting the left and right visual targets 8L, 8R, with the depth of focus of each subject eye EL, ER being increased by inserting the pinhole plate 50 into each of the corresponding visual-target projection systems 4. Therefore, the change in the objective refractive values (refraction values) obtained by the objective measurement portion 145 can be regarded as the convergence accommodation in response to the changes in the convergence distance Lc or the prism diopter set by the convergence position setting portion 144. Consequently, the calculation portion 146 determines, as convergence accommodation, the changes in the objective refractive values (refraction values) in response to the incrementally adjusted convergence distance Lc or the prism diopter.

Next, the process and flow for measuring the convergence accommodation in the convergence accommodation measuring portion 143 is described with reference to the flowchart shown in FIG. 6. The measurement process starts by selecting the mode to measure the convergence accommodation in the ophthalmologic apparatus 100.

In Step S1, a preliminary measurement is carried out, and then the process proceeds to Step S2. In Step S1, as the preliminary measurement, the main controller 141 obtains the eye characteristics including refractive values of the left and right subject eyes EL, ER based on the captured ring image and/or other relevant data. The preliminary measurement may be carried out two or more times, and the measurement conditions may be determined to be the same or different types at each preliminary measurement such that the measurement conditions converge as the measurements are repeated. Based on the obtained ring image, in each preliminary measurement, at least one of the measurement conditions is determined, such as the amount of light from the refraction measurement light source 61, the exposure time of the imaging element 59, the detection sensitivity of the imaging element 59, and the control details for the focusing lens 74.

In Step S2, a main measurement is carried out and then the process proceeds to Step S3. In Step S2, as the main measurement, the main controller 141 controls the measurement of the objective refractive values of the target left and right subject eyes EL, ER under the measurement conditions determined by the most recent preliminary measurement. The measurement of the objective refractive values of the left and right subject eyes EL, ER is carried out according to the following procedure. Firstly, the main controller 141 controls the projection of the ring-shaped light flux (infrared light) for the objective measurements onto the ocular fundi Elf, ERf of the left and right subject eyes EL, ER using the refraction measurement projection system 6. Then, the main controller 141 detects or receives the return light of the ring-shaped light flux from the ocular fundi Elf, ERf using the refraction measurement light receiving system 7, and obtains the objective refractive values of the left and right subject eyes EL, ER based on the image signals from the imaging element 59, using a known method.

In Step S3, the setup for starting the subjective examination is carried out, and then the process proceeds to Step S4. In Step S3, the main controller 141 sets up the start of the subjective examination for each of the left and right subject eyes EL, ER. In the setup for starting the subjective examination, based on the refractive values of the left and right subject eyes EL, ER obtained in the main measurement (Step S2) or the result of the RG test (Step S4), the position of the focusing lens 45 in the visual-target projection system 4 is adjusted so that the spherical power of the left and right subject eyes EL, ER is in a fully corrected condition when the presentation distance Lp is at the far-point vision position.

In Step S4, an RG test is carried out to determine whether or not it is in a fully corrected condition. If the condition is determined to be fully corrected (i.e., proper), the process proceeds to Step S5. If the condition is determined to be overcorrected or undercorrected, the process returns to Step S3. The RG test refers to a test that aims to subjectively check if the corrected condition of each of the left and right subject eyes EL, ER is the fully corrected condition (proper condition) (if it is over-corrected or under-corrected), using the RG chart, based on an optical characteristic known as chromatic aberration. The RG chart consists of several visual markers in different colors, such as red icons and green icons, which are arranged on the left and right respectively. For example, the visual markers may include numerical characters in different sizes; and/or a double-circled mark and/ a single-circled mark in different sizes as the visual targets 8. In the RG test, due to the characteristic of ocular media of the eye, in which a shorter-wavelength light has more power at the refractive surface, a green-wavelength light forms an image at a position that is rather shifted toward the incident side than a red-wavelength light. Thus, the under-corrected condition in the left and right subject eyes EL, ER, which causes the eyes to shift a focus point of the visual targets forward in front of the retina, and using a red-wavelength light, which causes a backward shift at the same time, allow the eyes to see the red visual target 8 more clearly. The over-corrected condition of the left and right subject eyes EL, ER causes the eyes to see the green visual target 8 more clearly. These mechanisms are used, in which the spherical powers (corrected powers) of the left and right subject eyes EL, ER are adjusted by changing the positions of the focusing lenses 45 by repeating Steps S3 to S4, until the examinee equally sees the green and red visual targets of the RG chart. As a result, the left and right subject eyes EL, ER can achieve the fully corrected values by the provisional values determined in the RG test.

Therefore, Steps S1 to S4 constitute the full correction setting steps to bring the left and right subject eyes EL, ER to a condition where the full correction values for the subjective examination are applied. However, the full correction values for the left and right subject eyes EL, ER may be any of the following values: values measured separately, prescription values of the current eyeglasses, or values determined through a complete subjective examination, rather than the provisional values from the RG test in the first embodiment. At this time, a phoria or strabismus examination may be carried out to correct the ocular alignment of the subject eyes EL, ER.

In Step S5, the left and right visual targets 8L, 8R are presented with an increased depth of focus, and then the process proceeds to Step S6. In Step S5, the convergence position setting portion 144 sets the initial prism angle in each of the left and right visual-target projection systems 4 of the measurement optical systems 125L, 125R, with the pinhole plate 50 inserted at the pupil conjugate position Q in the optical path, and presents the left-eye and right-eye visual targets 8L, 8R. In Step S5, the rotation angle control is carried out to set the initial prism angle. At this time, if the presentation distance Lp is close to the convergence distance Lc at the initial prism angle, the focusing lens is controlled to focus on any position (e.g., infinity).

In Step S6, the binocular fusion is checked, and then the process proceeds to Step S7. In Step S6, the convergence position setting portion 144 obtains a subjective response from the examinee regarding the binocular fusion of the left-eye and right-eye visual targets 8L, 8R. As the subjective response, for example, a response is obtained regarding whether the visual targets 8 appears as a single target due to the binocular fusion of the left-eye and right-eye visual targets 8L, 8R. If they are not seen as one, it is confirmed whether the examination is being properly carried out on the subject eyes. If the examination is confirmed to be improper, the settings are adjusted to achieve a proper condition. If the examination is confirmed to be proper, the examination may be stopped or continued after correcting the ocular alignment to make the targets appear as one.

In Step S7, a prism angle change process is carried out, and then the process proceeds to Step S8. In Step S7, while the pinhole plate 50 is inserted, the convergence position setting portion 144 incrementally changes the prism angle from 0Δ or the previous prism angle to the next prism angle by using the driver 122a. For example, the prism angle is changed in six stages: 0Δ, +1Δ, +2Δ, +4Δ, +6Δ, and +10Δ. For example, when the initial prism angle is 0Δ, it is changed in the following order: 0Δ, +1Δ, +2Δ, +4Δ, +6Δ, and +10Δ. In this example, the examination is carried out from a predetermined prism angle in a direction closer to the subject eye E, focusing only on a direction to check the adjustment. However, it is also possible to return to the predetermined prism angle after changing to a specified distance and then carry out the examination in the direction away from the subject eye E to determine whether there is an effect from factors such as strabismus or whether the adjustment changes when the convergence occurs in a divergence direction. The change of the prism angles is not limited to the above approach and may be adjusted as needed.

In Step S8, the binocular fusion is checked, and then the process proceeds to Step S9. In Step S8, as the convergence distance Lc changes incrementally, the convergence position setting portion 144 obtains a subjective response from the examinee regarding the binocular fusion of the left-eye and right-eye visual targets 8L, 8R. As the subjective response, for example, the convergence position setting portion 144 obtains a response regarding whether the visual targets 8 are perceived as a single target due to the binocular fusion between the left-eye and right-eye visual targets 8L, 8R. Therefore, in Step S8, when measuring the convergence accommodation of the left and right subject eyes EL, ER, it is possible to check if the subjective binocular fusion has been achieved.

In Step S9, the convergence position setting portion 144 determines whether or not the prism angle change process has been finished. If YES, the process proceeds to Step S13. If NO, the process returns to Step S7. In Step S9, the convergence position setting portion 144 determines whether or not the prism angle has been changed to the final prism angle (e.g., +10Δ). In other words, in Step S9, the convergence position setting portion 144 determines whether or not the measurement has been carried out for all the preset prim angles.

Steps S7 and S9 are convergence distance control steps that adjust the prism angle from the left and right subject eyes EL, ER toward the left-eye and right-eye visual targets 8L, 8R, thereby changing the convergence distance Lc between the left and right subject eyes EL, ER and the convergence position where the two lines of sight intersect. Steps S6 and S8 are subjective response obtaining steps that obtain the subjective response regarding the binocular fusion from the examinee when the left and right subject eyes EL, ER attempt the binocular fusion in response to the change of the convergence distance Lc.

In Step S10, the objective measurement portion 145 performs the refraction measurement. Specifically, the objective measurement portion 145 measures the eye characteristics of the left and right subject eyes EL, ER using the objective measurement optical system in response to the change in the convergence distance Lc and obtains the objective refractive values as the objective measurement information. In Step S11, the objective measurement portion 145 displays the refractive values at predetermined timings. Specifically, in Step S11, the objective measurement portion 145 displays the objective refractive values (refraction values) obtained at the timings when the prism angle changes. In Step S12, the objective measurement portion 145 compares the refraction values. Specifically, in Step S12, the objective measurement portion 145 compares the multiple refraction values obtained at the timings when the prism angle changes and calculates their average. Steps S10 to S12 are performed in parallel with the control of the convergence distance Lc. When the left and right subject eyes EL, ER attempt the binocular fusion in response to changes in the convergence distance Lc, the objective measurement optical system measures the eye characteristics of the left and right subject eyes EL, ER, thereby obtaining the objective refractive values as the objective measurement information.

In Step S13, the convergence accommodation is determined and the convergence accommodation measurement process is completed. In Step S13, the calculation portion 146 calculates the convergence accommodation (i.e., its value) based on the convergence distance Lc, i.e., the prism diopter that the convergence position setting portion 144 has adjusted, and the objective refractive value (average refraction value) from the objective measurement portion 145. Specifically, the convergence accommodation is the difference between the refractive value calculated from the presentation distance Lp (infinity in the first embodiment) and the objective refractive value obtained after changing the convergence distance Lc. The convergence accommodation may be determined as the difference using the objective refractive value(s) at one or more prism angles (convergence distance(s) Lc) at the measured positions. The convergence accommodation may be displayed either as values determined for each prism angle (convergence distance Lc) or as a graph showing its relationship with each prism angle.

Next, the flow of the convergence accommodation measurement process (each of the steps) is described. The convergence accommodation measurement process starts and proceeds in the order of Steps S1, S2, S3, and S4 once the examinee faces the ophthalmologic apparatus 100, positions his or her face at the predetermined location, and completes the alignment to properly adjust the positional relationship between the left and right subject eyes EL, ER, and the apparatus. After starting the process, the main controller 141 carry out the preliminary measurement, the main measurement, and the subjective examination start setting, and then sets the fully corrected value based on the provisional value in the RG test using the RG chart. Then, the measurement process proceeds to Steps S5 and S6. The convergence position setting portion 144 sets the initial prism angle with the pinhole plate 50 inserted and obtains a subjective response from the examinee regarding whether the binocular fusion is achieved.

Then, the measurement process proceeds to Steps S7, S8, and S9. The convergence position setting portion 144 incrementally changes the convergence distance Lc to correspond to each prism angle by changing from the initial prism angle (e.g., 0Δ) to the set prism angle. When the prism angle (convergence distance Lc) is changed, the subjective response is received from the examinee regarding whether the binocular fusion is achieved. This step is repeated until changing the prism angle (convergence distance Lc) to the last prism angle (e.g., +10Δ). In parallel with the change of the prism angle (convergence distance Lc), the flow of Steps S10, S11, and S12 is repeated, thereby allowing the objective measurement portion 145 to carry out the objective measurement and obtain the objective refractive value (refraction value) as the prism angle (convergence distance Lc) changes.

After completing the changes of all the set prism angles (convergence distances Lc), the process proceeds to Steps S9 and S13. The calculation portion 146 calculates the convergence accommodation (its value) of the left and right subject eyes EL, ER, based on the prism angle (convergence distance Lc), which has been changed by the convergence position setting portion 144, and the objective refractive value (average refraction value) from the objective measurement portion 145. Thus, in the flow of the convergence accommodation measurement process, the convergence distance control process and the objective measurement are executed in parallel. After the convergence distance control process is completed, the flow converges with the objective measurement at Step S13.

Here, the objective measurement portion 145 measures the objective refractive value (average refraction value) for each convergence distance Lc (prism angle). Since the calculation portion 146 determines the convergence accommodation based on the objective refractive value (average refraction value) for each convergence distance Lc (prism angle), it is possible to assess the convergence accommodation for various modes of change in the convergence distance Lc (prism angle).

Next, the operation for calculating the convergence accommodation is described with reference to FIG. 7. As mentioned above, firstly, the objective measurement portion 145 measures the eye characteristics of the left and right subject eyes EL, ER a predetermined number of times at each stage of the convergence distance Lc and obtains the average of the objective refractive values obtained the predetermined times at each stage. Thus, the objective measurement portion 145 can obtain the objective refractive value for each changed prism angle.

FIG. 7 shows the relationship between the prism angle and the objective refractive value in multiple examinees, where the average objective refractive value was obtained at each stage of the convergence distance Lc. The vertical axis indicates the objective refractive value, with each value measured in diopters (D). The horizontal axis indicates the prism angle, with each value measured in prism (Δ). The unit of prism may be converted to diopters (D) or the convergence angle (MA), expressed as the reciprocal of the convergence distance Lc.

The multiple examinees shown in FIG. 7 can be divided into a first group G1 and a second group G2 based on the relationship characteristics. The first group G1 shows a characteristic in which, when the prism angle is changed through six stages of 0Δ, +1Δ, +2Δ, +4Δ, +6Δ, and +10Δ, the objective refractive values tend to increase in response to the changing. Specifically, as the prism angle changes in the sequence of +2Δ, +4Δ, +6Δ, and +10Δ, the objective refractive value increases up to around 3.0 D.

Therefore, it is assumed that the left and right subject eyes EL, ER of the examinees in the first group G1 exhibit significant focus adjustment responses due to the convergence stimulus caused by changes in the convergence distance Lc. Consequently, it can be determined that the eyes of the examinees in the first group G1 have a strong tendency toward the convergence accommodation.

The second group G2 shows a characteristic in which, when the prism angle is changed through the six stages of "0Δ, +1Δ, +2Δ, +4Δ, +6Δ, and +10Δ", the objective refractive values remain constant and show no response to the changing. Specifically, even when the prism angle changes in the sequence of +2Δ, +4Δ, +6Δ, +10Δ, the objective refractive values remain low, generally less than 1.0 (D < 1.0).

Therefore, it is assumed that the left and right subject eyes EL, ER of the examinees in the second group G2 exhibit almost no focus adjustment responses, despite being subjected to the convergence stimulus caused by the changes in the convergence distance Lc. Consequently, it can be determined that the eyes of the examinees in the second group G2 have a weak tendency toward the convergence accommodation.

The ophthalmologic apparatus 100 of the first embodiment according to the present invention can achieve the following advantageous effects. The ophthalmologic apparatus 100 includes the display 41 as a visual-target presenting portion that is configured to present visual targets 8 to left and right subject eyes EL, ER, respectively; and an objective measurement optical system 6, 7 that is configured to objectively measure eye characteristics of the left and right subject eyes EL, ER, respectively. The visual-target presenting portion is configured to present the visual targets 8 while changing the convergence position R, where two lines of sight from positions of the left and right subject eyes EL, ER intersect, with the pinhole plates 50 respectively disposed at positions conjugate with pupils of the left and right subject eyes EL, ER. The objective measurement optical system 6, 7 is configured to obtain the objective refractive values of the left and right subject eyes E, respectively, under the condition where the pinhole plates 50 are respectively disposed by the visual-target presenting portion and the visual targets presented at the convergence position R are binocularly fused. Therefore, the ophthalmologic apparatus 100 can present the visual targets (left-eye visual target 8L and right-eye visual target 8R) while changing the convergence distance, with the depth of focus of the left and right subject eyes EL, ER increased, and can obtain the objective refractive values under such conditions. Accordingly, the ophthalmologic apparatus 100 can obtain the objective refractive values while providing only the stimuli for the convergence accommodation to the subject eyes EL, ER. This allows for the measurement of the convergence accommodation. Additionally, the ophthalmologic apparatus 100 enables the accurate determination of whether the eyes have a tendency toward the convergence accommodation.

The display 41 as the visual-target presenting portion is provided in a visual-target projection system 4 that projects the visual targets onto the left and right subject eyes EL, ER, respectively. Accordingly, the ophthalmologic apparatus 100 can present the visual targets 8 with the left and right subject eyes EL, ER in a condition of increased depth of focus, using a simple configuration where the pinhole plate 50 is merely inserted at the pupil conjugate position Q in the optical path of the visual-target projection system 4.

The ophthalmologic apparatus 100 further includes a left measuring head portion 122L including a left-side visual-target projection system (visual-target projection system 4) and a left-side objective measurement optical system (objective measurement optical system 6, 7), both aligned on the same optical axis to correspond to the left subject eye EL. The ophthalmologic apparatus 100 further includes a right measuring head portion 122R including a right-side visual-target projection system (visual-target projection system 4) and a right-side objective measurement optical system (objective measurement optical system 6, 7), both aligned on the same optical axis to correspond to the right subject eye ER. The visual-target presenting portion is configured to change the convergence position R where the visual targets 8 are presented by rotating the left and right measuring head portions 122L, 122R by equal angles in opposite directions, with the pinhole plates 50 respectively disposed in the left-side and right-side visual-target projection systems. Therefore, the ophthalmologic apparatus 100 can present the visual targets in a condition where the depth of focus of the left and right subject eyes EL, ER has been increased, while changing the convergence position R with a simple configuration. The ophthalmologic apparatus 100 can perform simultaneous parallel processing to present the visual targets 8 in a condition where the depth of focus of the left and right subject eyes EL, ER has been increased, while changing the convergence position R, and to obtain the objective refractive values of the left and right subject eyes EL, ER. This allows for a more simplified measurement of the convergence accommodation. Furthermore, when changing the convergence position R through the rotation control of the left and right measuring head portions 122L, 122R, the ophthalmologic apparatus 100 can maintain a condition in which the visual axes from the left and right subject eyes EL, ER coincide with the optical axes of the left and right measurement optical systems 125L, 125R. Therefore, due to the coincidence of the visual axes with the optical axes, the ophthalmologic apparatus 100 can measure ocular alignment and the direction of the line of sight to verify whether the left and right subject eyes EL, ER have achieved the objective binocular vision, based on the anterior segment image.

The left-side and right-side objective measurement optical systems (objective measurement optical systems 6, 7) of the ophthalmologic apparatus 100 are configured to obtain the objective refractive values of the left and right subject eyes EL, ER, respectively, each time the left and right measuring head portions 122L, 122R are rotated. Therefore, the ophthalmologic apparatus 100 can easily obtain the objective refractive values of the left and right subject eyes EL, ER with a simple configuration, under the condition where the visual targets 8 are presented with the increased depth of focus while changing convergence position R, that is, the condition where only the convergence stimulus is provided.

The ophthalmologic apparatus 100 further includes a controller 140 that is configured to control the visual-target projection system 4 and the objective measurement optical systems 6, 7. The controller 140 is further configured to determine the convergence accommodation based on the changes in the objective refractive values relative to the changes in the convergence position R. Therefore, the ophthalmologic apparatus 100 can determine the convergence accommodation in response to the change in the objective refractive values.

In addition, the ophthalmologic apparatus 100 of the first embodiment carries out the full correction setting steps (Steps S1 to S4) prior to presenting the visual targets 8, with the left and right subject eyes EL, ER having the increased depth of focus while changing the convergence distance Lc. Therefore, the ophthalmologic apparatus 100 presents the visual targets 8 under conditions where the left and right subject eyes EL, ER have the increased depth of focus while changing the convergence distance Lc, based on a reference where the visual targets 8 are clearly seen with the set fully corrected values. This allows for a smoother and more accurate transition to the measurement of the convergence accommodation.

Therefore, the ophthalmologic apparatus of the first embodiment according to the present invention can properly measure the convergence accommodation.

The ophthalmologic apparatus of the present invention has been described based on the first embodiment. However, the specific configurations of the ophthalmologic apparatus are not limited to those in the first embodiment. Design changes, additions, and modifications are permitted as long as they do not depart from the scope of the inventions as defined in the claims.

The ophthalmologic apparatus 100 of the first embodiment is an example of an objective measurement device that includes subjective examination functions using internal visual targets, as well as a visual-target presenting function, a phoropter function, and an automatic refraction and keratometry measurement function. However, the ophthalmologic apparatus is not limited to the configuration of the first embodiment. The ophthalmologic apparatus may, for example, be a combined device consisting of a visual projection apparatus that sets a condition in which the convergence distance is changed while presenting real visual targets through pinholes, and an apparatus equipped with a phoropter function and an automatic refraction measurement function.

The ophthalmologic apparatus 100 of the first embodiment controls the change in the sight angle (prism angle) by controlling the change in the rotation angle for rotating the left and right measuring head portions 122L, 122R in opposite directions. However, the ophthalmologic apparatus is not limited to the control of the sight angle in the first embodiment. For example, the ophthalmologic apparatus may control the change in the sight angle (prism angle) by inserting a prism lens at a position on the optical axis between the left and right subject eyes and the visual-target presenting portion of the visual-target projection system. **In** addition, as long as it presents the visual target 8 to the subject eye E, the visual-target presenting portion may be, for example, a real target or a display panel placed in front of the examinee, instead of being provided in the visual-target projection system. Even in this case, it is possible to measure the convergence accommodation in the same way as described above by changing the sight angle (prism angle) through the insertion of a prism lens at a position on the optical axis between the left and right subject eyes and the visual-target presenting portion. The convergence accommodation may be measured in the same way as described above by presenting a stereo image with a change in the convergence distance, while keeping the presentation distance constant, in the visual-target presenting portion using a 3D display or similar device.

Furthermore, the ophthalmologic apparatus 100 of the first embodiment may automatically perform alignment if the alignment becomes misaligned while the objective measurement optical systems 6, 7 are measuring the objective refractive values. This can be achieved by carrying out alignment as described above when the controller 140 controls the anterior ocular segment observation system 5 to continuously obtain anterior ocular segment images while controlling the objective measurement optical systems 6, 7 to carry out the measurement and determines that the bright spot image is displaced from the center of the alignment mark or that the ratio of the interval between the two spot images to the diameter of the kerato-ring image falls outside a predetermined range.

As mentioned above, the ophthalmologic apparatus 100 of the first embodiment determines the convergence accommodation by using the objective refractive values corresponding to each change in the prism angle. However, the ophthalmologic apparatus 100 may obtain the objective refractive values sequentially (continuously) and determine the convergence accommodation based on the moment when the convergence position R changes and the subsequent changes. **In** this case, the convergence accommodation can be classified into an instance where the accommodation is continuously active due to convergence, an instance where the accommodation is momentarily active but then returns to its original state, and an instance where no accommodation occurs (or the accommodation does not take effect).

As mentioned above, the ophthalmologic apparatus 100 of the first embodiment determines the convergence accommodation by using the objective refractive values obtained for each change in the prism angle. However, the ophthalmologic apparatus 100 may also determine whether miosis, as one of the near viewing responses, occurs due to convergence. This can be measured by continuously obtaining the anterior ocular segment images with the anterior ocular segment observation system 5 while the objective measurement optical system 6, 7 is carrying out the measurement, and then by determining the size of the pupil in the anterior ocular segment images. The pupil size may be determined either by analyzing the anterior ocular segment image using the controller 140 or by having the examiner observe the anterior ocular segment images displayed on the display 131.

### List of Reference Signs

- 100: ophthalmologic apparatus
- 4: visual target projection system
- 8: visual target
- 41: display (an example of visual-target presenting portion)
- 6, 7: objective measurement optical systems
- 50: pinhole plate (an example of pinhole)
- 122L: left measuring head portion
- 122R: right measuring head portion
- 140: controller
- E: subject eye
- R: convergence position

## Claims

1. An ophthalmologic apparatus (100) comprising:
a visual-target presenting portion (41) that is configured to present visual targets (8) to left and right subject eyes (EL, ER), respectively; and
an objective measurement optical system (6, 7) that is configured to objectively measure eye characteristics of the left and right subject eyes (EL, ER), respectively,
wherein the visual-target presenting portion (41) is configured to present the visual targets (8) while changing a convergence position (R, R1, R2), where two lines of sight (SL1, SR1, SL2, SR2) from positions of the left and right subject eyes (EL, ER) intersect, with pinhole plates (50) respectively disposed at positions conjugate with pupils of the left and right subject eyes (EL, ER), and
wherein the objective measurement optical system (6, 7) is configured to obtain the objective refractive values of the left and right subject eyes (EL, ER), respectively, under a condition where the pinhole plates (50) are respectively disposed by the visual-target presenting portion (41) and the visual targets (8) presented at the convergence position (R, R1, R2) are binocularly fused.

2. The ophthalmologic apparatus (100) according to claim 1, wherein the visual-target presenting portion (41) is provided in a visual-target projection system (4) that is configured to project the visual targets (8) onto the left and right subject eyes (EL, ER), respectively.

3. The ophthalmologic apparatus (100) according to claim 2, further comprising:
a left measuring head portion (122L) comprising a left-side visual-target projection system (4) and a left-side objective measurement optical system (6, 7), both aligned on a common optical axis to correspond to the left subject eye (EL), and
a right measuring head portion (122R) comprising a right-side visual-target projection system (4) and a right-side objective measurement optical system (6, 7), both aligned on a common optical axis to correspond to the right subject eye (ER),
wherein the visual-target presenting portion (41) is configured to change the convergence position (R, R1, R2) where the visual targets (8) are presented by rotating the left and right measuring head portions (122L, 122R) by equal angles in opposite directions, with the pinhole plates (50) respectively disposed in the left-side and right-side visual-target projection systems (4).

4. The ophthalmologic apparatus (100) according to claim 3, wherein the left-side and right-side objective measurement optical systems (6, 7) are configured to obtain the objective refractive values of the left and right subject eyes (EL, ER), respectively, each time the left and right measuring head portions (122L, 122R) are rotated.

5. The ophthalmologic apparatus (100) according to one of claims 2 to 4, further comprising a controller (140) that is configured to control the visual-target projection system (4) and the objective measurement optical system (6, 7),
wherein the controller (140) is further configured to determine a convergence accommodation based on a change in the objective refractive values relative to a change in the convergence position (R, R1, R2).

## Patentansprüche

1. Ophthalmologische Vorrichtung (100) umfassend:
einen visuellen Zielpräsentationsabschnitt (41), der konfiguriert ist, um visuelle Ziele (8) dem linken bzw. rechten Patientenauge (EL, ER) zu präsentieren; und
ein objektives optisches Messsystem (6, 7), das konfiguriert ist, um Augenmerkmale des linken und rechten Patientenauges (EL, ER) objektiv zu messen,
wobei der visuelle Zielpräsentationsabschnitt (41) konfiguriert ist, um die visuellen Ziele (8) während des Änderns einer Konvergenzposition (R, R1, R2), in der sich zwei Sichtlinien (SL1, SR1, SL2, SR2) von Positionen des linken und rechten Patientenauges (EL, ER) überschneiden, zu präsentieren, wobei Lochblendenplatten (50) jeweils an Positionen angeordnet sind, die mit den Pupillen des linken und des rechten Patientenauges (EL, ER) konjugiert sind, und
wobei das objektive optische Messsystem (6, 7) konfiguriert ist, um jeweils die objektiven Refraktionswerte des linken und rechten Patientenauges (EL, ER) unter einer Bedingung zu erhalten, bei der die Lochblendenplatten (50) jeweils durch den visuellen Zielpräsentationsabschnitt (41) angeordnet sind und die an der Konvergenzposition (R, R1, R2) präsentierten visuellen Ziele (8) binokular fusioniert sind.

2. Ophthalmologische Vorrichtung (100) nach Anspruch 1, wobei der visuelle Zielpräsentationsabschnitt (41) in einem visuellen Zielprojektionssystem (4) vorgesehen ist, das konfiguriert ist, um die visuellen Ziele (8) auf das linke bzw. rechte Patientenauge (EL, ER) zu projizieren.

3. Ophthalmologische Vorrichtung (100) nach Anspruch 2, ferner umfassend:
einen linken Messkopfabschnitt (122L), der ein linksseitiges visuelles Zielprojektionssystem (4) und ein linksseitiges objektives optisches Messsystem (6, 7) umfasst, die beide auf einer gemeinsamen optischen Achse ausgerichtet sind, um dem linken Patientenauge (EL) zu entsprechen, und
einen rechten Messkopfabschnitt (122R), der ein rechtsseitiges visuelles Zielprojektionssystem (4) und ein rechtsseitiges objektives optisches Messsystem (6, 7) umfasst, die beide auf einer gemeinsamen optischen Achse ausgerichtet sind, um dem rechten Patientenauge (ER) zu entsprechen,
wobei der visuelle Zielpräsentationsabschnitt (41) konfiguriert ist, um die Konvergenzposition (R, R1, R2), in der die visuellen Ziele (8) präsentiert werden, durch Drehen des linken und rechten Messkopfabschnitts (122L, 122R) um gleiche Winkel in entgegengesetzte Richtungen zu ändern, wobei die Lochblendenplatten (50) jeweils in den linksseitigen und rechtsseitigen visuellen Zielprojektionssystemen (4) angeordnet sind.

4. Ophthalmologische Vorrichtung (100) nach Anspruch 3, wobei die linksseitigen und rechtsseitigen objektiven optischen Messsysteme (6, 7) konfiguriert sind, um jeweils die objektiven Refraktionswerte des linken und rechten Patientenauges (EL, ER) immer dann zu erhalten, wenn der linke und der rechte Messkopfabschnitt (122L, 122R) gedreht werden.

5. Ophthalmologische Vorrichtung (100) nach einem der Ansprüche 2 bis 4, ferner umfassend eine Steuereinheit (140), die konfiguriert ist, um das visuelle Zielprojektionssystem (4) und das objektive optische Messsystem (6, 7) zu steuern,
wobei die Steuereinheit (140) ferner konfiguriert ist, um eine Konvergenzakkommodation basierend auf einer Änderung der objektiven Refraktionswerte relativ zu einer Änderung der Konvergenzposition (R, R1, R2) zu bestimmen.

## Revendications

1. Appareil ophtalmologique (100) comprenant:
une partie de présentation des cibles visuelles (41) configurée pour présenter des cibles visuelles (8) aux yeux gauche et droit du sujet (EL, ER), respectivement ; et
un système optique de mesure objective (6, 7) configuré pour mesurer objectivement des caractéristiques oculaires des yeux gauche et droit du sujet (EL, ER), respectivement,
dans lequel la partie de présentation des cibles visuelles (41) est configurée pour présenter les cibles visuelles (8) tout en changeant une position de convergence (R, R1, R2), où deux lignes de visée (SL1, SR1, SL2, SR2) provenant des positions des yeux gauche et droit du sujet (EL, ER) se croisent, avec des plaques à sténopé (50) respectivement disposées à des positions conjuguées avec les pupilles des yeux gauche et droit du sujet (EL, ER), et
dans lequel le système optique de mesure objective (6, 7) est configuré pour obtenir les valeurs objectives de réfraction des yeux gauche et droit du sujet (EL, ER), respectivement, dans une condition où les plaques à sténopé (50) sont respectivement disposées par la partie de présentation des cibles visuelles (41) et où les cibles visuelles (8) présentées à la position de convergence (R, R1, R2) sont fusionnées de manière binoculaire.

2. Appareil ophtalmologique (100) selon la revendication 1, dans lequel la partie de présentation des cibles visuelles (41) est prévue dans un système de projection des cibles visuelles (4) qui est configuré pour projeter les cibles visuelles (8) respectivement sur les yeux gauche et droit du sujet (EL, ER).

3. Appareil ophtalmique (100) selon la revendication 2, comprenant en outre:
une partie de tête de mesure gauche (122L) comprenant un système de projection des cibles visuelles du côté gauche (4) et un système optique de mesure objective du côté gauche (6, 7), tous deux alignés sur un axe optique commun pour correspondre à l'œil gauche du sujet (EL), et
une partie de tête de mesure droite (122R) comprenant un système de projection des cibles visuelles du côté droit (4) et un système optique de mesure objective du côté droit (6, 7), tous deux alignés sur un axe optique commun pour correspondre à l'œil droit du sujet (ER),
dans lequel la partie de présentation des cibles visuelles (41) est configurée pour changer la position de convergence (R, R1, R2) dans laquelle les cibles visuelles (8) sont présentées en faisant tourner les parties de tête de mesure gauche et droite (122L, 122R) d'un angle égal dans des directions opposées, avec les plaques à sténopé (50) respectivement disposées dans les systèmes de projection des cibles visuelles (4) du côté gauche et du côté droit.

4. Appareil ophtalmologique (100) selon la revendication 3, dans lequel les systèmes optiques de mesure objective du côté gauche et du côté droit (6, 7) sont configurés pour obtenir les valeurs objectives de réfraction des yeux gauche et droit du sujet (EL, ER), respectivement, chaque fois que les parties de tête de mesure gauche et droite (122L, 122R) sont tournées.

5. Appareil ophtalmologique (100) selon l'une des revendications 2 à 4, comprenant en outre un contrôleur (140) qui est configuré pour contrôler le système de projection des cibles visuelles (4) et le système optique de mesure objective (6, 7),
dans lequel le contrôleur (140) est en outre configuré pour déterminer une accommodation de convergence sur la base d'un changement des valeurs de réfraction objectives par rapport à un changement de la position de convergence (R, R1, R2).
